**Europäisches Patentamt**

**European Patent Office**

**Office européen des brevets**

(19)

(11) Publication number: **0 349 257**
**A1**

## EUROPEAN PATENT APPLICATION

(21) Application number: **89306495.6**

(22) Date of filing: **27.06.89**

(51) Int. Cl.⁴: **G01N 33/577 , G01N 33/68 , C12P 21/00 , C12Q 1/68**

(30) Priority: **28.06.88 JP 162223/88**

(43) Date of publication of application:
**03.01.90 Bulletin 90/01**

(84) Designated Contracting States:
**CH DE FR GB IT LI SE**

(71) Applicant: **Shin-Etsu Chemical Co., Ltd.**
**6-1, Otemachi 2-chome**
**Chiyoda-ku Tokyo 100(JP)**

(72) Inventor: **Onodera, Kazukiyo**
**7-27-314, Hikawadai 2-chome Nerima-ku**
**Tokyo(JP)**
Inventor: **Obata, Ranko**
**1-599, Kosugi Jinya-Machi Nakahara-ku**
**Kawasaki-shi Kanagawa-ken(JP)**
Inventor: **Ito, Shinichi**
**Magnolia Court Woodside Grange Road N12**
**London(GB)**

(74) Representative: **Votier, Sidney David et al**
**CARPMAELS & RANSFORD 43, Bloomsbury**
**Square**
**London WC1A 2RA(GB)**

(54) Method of diagnosing down's disease.

(57) A method of diagnosing Down's disease comprising the step of applying a particular monoclonal antibody to a human serum. The monoclonal antibody has been produced by a hybridoma obtained by immunizing an animal with a Chinese hamster ovary cell which expresses at least one membrane protein encoded by human chromosome No. 21 and fusing an antibody-producing cell taken from said animal with a myeloma cell to form hybridomas, and has a characteristic that it specifically binds said Chinese hamster ovary cell but does not bind a normal Chinese hamster ovary cell. This method is useful in the diagnosis of Down's disease.

EP 0 349 257 A1

EP 0 349 257 A1

**Method of diagnosing Down's disease**

BACKGROUND OF THE INVENTION

1. Field of the Invention

This invention relates to a method of diagnosing Down's disease, and particularly to a method of diagnosing Down's disease using a monoclonal antibody capable of recognizing a serum protein inherent in Down's disease patients.

2. Description of the Prior Art

Down disease is related to an aberration in chromosome number, called trisomy-21. Since the Down disease patients were liable to suffer from infections, leukemia and the like in 1960s, their average life span was 20 years or less. In recent years, the average life span of Down disease patiants became over 50 years because of the development of new medicines including antibiotics and the development of medical technology as well as the improvement of nutrition.

It is desired to be able to expect the possibility of an attack with Down disease and to give a suitable treatment to a patient in early stages in Down disease. However, no methods or means capable of easily diagnosing Down's disease or its conditions is heretofore known.

SUMMARY OF THE INVENTION

Accordingly, an object of this invention is to provide a method of easily diagnosing Down's disease and its condition.

Thus, this invention provides a method of diagnosing Down's disease comprising the step of applying a monoclonal antibody

that has been produced by a hybridoma obtained by a process comprising the steps of immunizing an animal with a Chinese hamster ovary cell into which human chromosome No. 21 has been introduced such that said cell expresses at least one membrane protein encoded by human chromosome No. 21 and fusing an antibody-producing cell taken from said animal with a myeloma cell to form hybridomas, and

that specifically binds said Chinese hamster ovary cell that expresses at least one membrane protein encoded by human chromosome No. 21 but does not bind a normal Chinese hamster ovary cell, to a human serum.

The monoclonal antibody used in this invention binds a protein that is not present in serums of normal human being but is present inherently in the serums of Down's disease patients. Therefore, the method of this invention is useful in diagnosis of Down's disease and its condition, and also useful in diagnosis of the possibility that a Down's disease patient may be attack with Alzheimer's disease in the future, as described later.

DETAILED DESCRIPTION OF THE PREFERRED EMBODIMENTS

The monoclonal antibody used in this invention is disclosed in the Japanese Unexamined Patent Publication (KOKAI) No. 240,797/1988. This monoclonal antibody can be obtained by a process comprising the steps of immunizing an animal with a Chinese hamster ovary (CHO) cell into which human chromosome No. 21 has been introduced and which synthesizes a membrane protein encoded by human chromosome No. 21; fusing an antibody-producing cell taken from the animal with a myeloma cell to form hybridomas; subjecting the hybridomas to selection, screening and cloning to obtain a hybridoma producing a desired monoclonal antibody; and culturing the hybridoma to obtain such monoclonal antibody.

The above screening procedure is carried out using the characteristic of the monoclonal antibody used in this invention that the monoclonal antibody can bind the CHO cell used in the above immunization but does not bind normal CHO cells.

The CHO cell into which human chromosome NO. 21 has been introduced so that the cell expresses a

2

membrane protein encoded by human chromosome No. 21 can be obtained by known cell fusion techniques. An example of such a CHO cell is a cell of the so-called 2Fu$^r$ line. [see D. Patterson et al., Som. Cell Genet. 1; 91-110 (1975)].

Examples of animals which are suitable to be immunized include rodents like mouse and rat. Mice are usually used in this regard.

Route and schedule of administration of the antigen into the animal may variously be varied. According to a preferred administration method, for example, the antigen is first treated with mitomycin and intraperitoneally administered. Antibodies are produced in the spleen of the animal as an immunological response to administration of the antigen. As antibody-producing cells, spleen cells are usually used.

For the myeloma cell to be fused with the antibody-producing cells thus obtained, a cell derived from BALB/C mouse MOPC 21 myeloma (P3-NS1-Ag4-1) can be used. The myeloma P3-NS1-Ag4-1 is resistant to 8-azaguanine, and dies in a medium containing hypoxanthine-aminopterinthymidine (HAT medium) because it lacks the enzyme hypoxanthine guanine phosphoribosyltransferase. Therefore, use of P3-NS1-Ag4-1 is convenient for selective culture of hybridomas to be obtained. Polyethylene glycol is typically used as a fusing agent for cell fusion. The above-mentioned cell fusion techniques, myeloma cells to be used, and the like are known [meyloma cells: Köhler et al., Eur. J. Immunol. 6: 292-295 (1976); fusing method: Köhler et al., ibid. 6: 511-516 (1976)].

The resulting hybridomas are screened to select those which can produce antibodies capable of binding the CHO cell used for the immunization stated above (i.e., the CHO cell into which human chromosome No. 21 has been introduced and expresses a membrane protein encoded by the human chromosome No. 21) but incapable of binding a normal CHO cell. The hybridomas thus screened are then cloned, whereby progeny of different hybridomas from respective phyletic lines are obtained. Monoclonal antibodies that can recognize a membrane protein chracteristic of cells where chromosome No. 21 is present in aberrant number can be synthesized and secreted by culturing the hybridomas to produce cell lines, i.e., by propagating each clone in a cell culture medium (in vitro culture) or in vivo. Each of the monoclonal antibodies thus produced can be recovered according to a method known in the art.

The monoclonal antibody used in this invention is also, as described in the Japanese Unexamined Patent Publication (KOKAI) No. 240,797/1988, characterized in that it specifically binds a human fibroblast cell with a membrane protein component encoded by chromosome No. 21 in an aberrant number but does not bind a normal human fibroblast cell.

It has also been found that the monoclonal antibody used in this invention specifically recognizes and binds a protein which is inherent in the serums of Down's disease patients but is not present in serums of normal human beings at least at a detectable concentration.

Consequently, said monoclonal antibody is useful in diagnosis of Down's disease and its condition by applying it to human serums.

Almost all the Down's disease patients give pathologic findings similar to those in the case of Alzheimer's disease at their age of 30 to 40. It has been recently proved that the gene coding for amyloid protein characteristic of brains of Alzheimer's disease patients is located in chromosome No. 21. Further, it is said that some Alzheimer's disease patients may have an aberration in chromosome No. 21 which is different from trisomy. In view of these, the mothod of diagnosis of this invention is expected to be useful in diagonsing the process in which a Down's disease patient is attacked with Alzheimer's disease.

The method of this invention can be conducted in practice by using any immunological test method, for example, immunofluorescence, an immunoadhesionhemocyte-agglutinating reaction method, radioimmunoassay or the like.

The present invention will now be described in more detail with reference to Examples.

## EXAMPLES

Preparation example (Preparation of Monoclonal Antibody)

(1) Procuction of antibodies

CHO cells (2Fu$^r$) into which human chromosome No. 21 had been introduced and which synthesized a membrane protein encoded by the chromosome No. 21 were treated with mitomycin C and then washed with PBS (phosphate buffer). A 2Fu$^r$ suspension of 5 x 10$^6$ cells/0.5 cc in PBS was intraperitoneally injected into a female BALB/C mouse (immunization). Two weeks thereafter, a 2Fu$^r$ suspension of 1 x 10$^7$ cells/0.5 cc in PBS, and further two weeks thereafter, a 2Fu$^r$ suspension of 1 x 10$^7$ cells/0.5 cc in PBS were injected in the same manner as above. Three days thereafter, the spleen of the mouse was taken out, finely pulverized in RPMI-1640 medium, subjected three times to centrifugation (1500 rpm) for washing, and resuspended in the medium.

Sample cultures of 2Fu$^r$ used here can be obtained upon written request to Shin-Etsu Chemical Co., Ltd., 6-1, Ohtemachi 2-chome, Chiyoda-ku, Tokyo, Japan.

(2) Cell fusion

1.1 x 10$^8$ cells of the spleen cell obtained in the above procedure and 1.0 x 10$^7$ cells of BALB/C mouse myeloma cell (P3-NS1-Ag4-1) once washed in advance with RPMI-1640 medium were mixed, and centrifuged at 1500 rpm for 5 minutes to prepare a pellet. Then, 1 ml of a solution obtained by dissolving polyethylene glycol (1540) in RPMI-1640 medium to 50 wt% was added to the pellet with stirring, RPMI-1640 medium was added thereto to the total volume of 10 ml, and the mixture was stirred for 6 minutes. The mixture was subjected to centrifugation at 1000 rpm for 5 minutes to remove polyethylene glycol, and the solid matters were resuspended in 40 ml of RPMI-1640 medium containing 20 vol% of fetal calf serum (FCS).

(3) Selection, screening and cloning

0.2 ml (5 x 10$^6$ cells) of the above suspension was placed in each of 192 wells of a 96 wells-plate for tissue culture, and half amount of the culture supernatant was replaced every 3 to 4 days with HAT medium (136.1 mg/ml hypoxanthine, 1.76 mg/ml aminopterine and 38.75 mg/ml thymidine). The culturing condition was a temperature of 37°C, a humidity of 100% and a $CO_2$ gas concentration of 7%. 10 days after the start of the culturing, hybridomas were observed in 119 wells (emergence rate: 62%). Culture supernatants in the 119 wells, which revealed positive results, were subjected to screening, by means of an enzyme-labeled antibody technique (ELISA) using 2Fu$^r$, to determine whether the supernatants contained antibodies. The test results indicated that 20 wells (10.6% of the total number) were positive.

Cells in each of the 20 wells were subjected to limiting dilution using HAT medium with supplementation of BALB/C mouse thymocyte (1.0 x 10$^7$ cells/ml), 0.2 ml portions of the resulting cell suspensions were placed in each well of a 96 wells-plate for tissue culture, and culturing was carried out. In this connection, hybridoma number per well and well number were 48 wells of 1 cell/well, 45 wells of 5 cells/well and 3 cells of 20 cells/well. Culturing conditions were the same as above. The culture supernatants were respectively subjected to the same ELISA as above-mentioned to screen those which are negative for the CHO cell and positive for 2Fu$^r$, and as the result desired clones were obtained from three wells. The cells in each of the three wells were recloned two times according to the limiting dilution method, and three kinds of resulting hybridomas were named OK-1, OK-2 and OK-3. Sample cultures of hybridomas OK-2 and OK-3 are available from FRI (Fermentation Research Institute Agency of Industrial Science and Technology), Ibaraki, Japan under the deposit Nos. FERM BP-1802 and FERM BP-1803, respectively.

It has been found by an enzyme-labeled antibody technique using an antimouse antibody that monoclonal antibodies produced from hybridomas OK-1, OK-2 and OK-3 belong to the classes IgM, IgG and IgM, respectively.

Furthermore, proteins to which the monoclonal antibodies produced by OK-1, OK-2 and OK-3 respectively recognize and bind are as follows.

- Monoclonal antibody produced by OK-1

This antibody recognizes four bands in Western blotting of human leukemia cell (TALL-1) protein.

- Monoclonal antibody produced by OK-2

This antibody recognizes bands of 40 and 90 kilodaltons in Western blotting of TALL-1 protein.

- Monoclonal antibody produced by OK-3

This antibody recognizes four bands of 86.4, 74.2, 61.4 and 36.4 kilodaltons in Western blotting of TALL-1 protein, and recognizes three bands of 89, 82 an 45 kilodaltons in Western blotting of 2Fu$^r$ protein.

Note, Tall-1 cell line: see MAMMALIAN CELL CULTURE TECHNOLOGY, edited by M. Shikita et al. SOFT SCIENCE PUBLICATIONS, Tokyo, pp.141-162, 1985.

<u>Example</u>

<u>Detection of a serum protein inherent in Down's disease patients by Western blotting</u>

Serums were separated from nine Down's disease patients and six normal human beings, and each of them is subjected to SDS-polyacrylamide-gel electrophoresis (Concentration of acrylamide, concentrated gel: 3%, separated gel: 10%). In all the electrophoreses, the protein was added to each well of the gel in an amount of 20μg. After the electropheresis, the gel was immersed for 15 minutes in a blotting buffer (25 mM tris-HCl, 192 mM glycine and 20% methanol), and the protein bands in the gel were then transfered to a sheet of nitrocellulose paper by carrying out electroblotting at 30 volt overnight. The nitrocellose sheet was treated at 4°C in a blocking solution (1% skim milk, 20mM tris-HCl, 100mM, NaCl, pH 7.6). Thereafter, the nitrocellose sheet was immersed and treated with shaking at room temperature for 12 hours in a solution prepared by diluting the monoclonal antibody produced by the hybridoma OK-2 to 400-fold with the blocking solution (primary antibody treatment).

The nitrocellose sheet was washed by immersing it in TBST (50mM tris-HCl, 200mM NaCl, 0.1% Tween 20) for 20 minutes. This washing operation was repeated three times. After washed, the nitrocellose sheet was allowed to react for one hour in a blocking solution containing 10μg/ml of biotin-bound anti-mouse IgG (secondary antibody treatment). The nitrocellose was washed three times by immersing it in TBST for 20 minutes each time, and thereafter avidinbiotin-bound horseradish peroxidase complex was added to the nitrocellose sheet to allow them to react at room temperature for 10 minutes. The nitrocellose sheet was then washed with TBST three time, by immersing it in TBST for 20 minutes each time, and further washed with TBS (20mM tris-Hcl, 100mM Nacl, pH 7.6) three times by immersing it in TBS for 20 minutes each time.

The nitrocellose sheet was then immersed in a color producing solution (alpha-1,4-chloronaphthol (0.5mg/ml), an aromatic amine compound (0.2mg/ml), 0.01% hydrogen peroxide), and the protein bands were thereby color-produced.

The results obtained are given in Table 1.

Table 1

| Frequency of detection of serum protein inherent in Down's disease patients | | |
|---|---|---|
| Sample | Protein that the monoclonal antibody produced by OK-2 recognizes | |
| | 40 | 90 |
| Serums of Down's disease patients | 8/9 | 8/9 |
| Serums of normal human beings | 0/6 | 4/6 |

The results described above show that the monoclonal antibody produced by OK-2 binds the 40 kd protein but that this 40 kd protein is not present in the serums of normal human beings. That is, the 40 kd protein is considered to be encoded by chromosome No. 21 and to be present in the serums from Down's disease patients.

**Claims**

1. A method of diagnosing Down's disease comprising the step of applying a monoclonal antibody

that has been produced by a hybridoma obtained by a process comprising the steps of immunizing an animal with a Chinese hamster ovary cell into which human chromosome No. 21 has been introduced such that said cell expresses at least one membrane protein encoded by human chromosome No. 21 and fusing an antibody-producing cell taken from said animal with a myeloma cell to form hybridomas, and

that specifically binds said Chinese hamster ovary cell that expresses at least one membrane protein encoded human chromosome No. 21 but does not bind a normal Chinese hamster ovary cell,

to a human serum.

2. The method of Claim 1, wherein said monoclonal antibody is produced by a cell line OK-2 or OK-3.

3. For use in diagnosing Down's disease, a monoclonal antibody

that has been produced by a hybridoma obtained by a process comprising the steps of immunizing an animal with a Chinese hamster ovary cell into which human chromosome No. 21 has been introduced such that said cell expresses at least one membrane protein encoded by human chromosome No. 21 and fusing an antibody-producing cell taken from said animal with a myeloma cell to form hybridomas, and

that specifically binds said Chinese hamster ovary cell that expresses at least one membrane protein encoded human chromosome No. 21 but does not bind a normal Chinese hamster ovary cell.

4. The monoclonal antibody of Claim 3 which is produced by a cell line OK-2 or OK-3.

| Category | Citation of document with indication, where appropriate, of relevant passages | Relevant to claim | CLASSIFICATION OF THE APPLICATION (Int. Cl.4) |
|---|---|---|---|
| | | | EP 89306495.6 |
| A | CHEMICAL ABSTRACTS, vol. 103, no. 11, September 16, 1985, Columbus, Ohio, USA J.N. DAVIDSON et al. "The isolation of gene sequences on human chromosome 21." page 148, abstract no. 82 613j & ICSU Short Rep. 1984, 1 (Adv. Gene Technol.), 148-9 -- | 1,3 | G 01 N 33/577 G 01 N 33/68 C 12 P 21/00 C 12 Q 1/68 |
| A | CHEMICAL ABSTRACTS, vol. 97, no. 15, October 11, 1982, Columbus, Ohio, USA M.L. VAN KEUREN et al.: "A quantitative two-dimensional electrophoretic survey of proteins affected by chromosome 21." page 527, abstract no. 125 335y & Electrophor. '81 (Eighty-One), Proc. Int. Conf., 3rd 1981, 355-69 -- | 1,3 | |
| A | WO - A1 - 86/03 227 (DGI, INC.) * Abstract; page 9, lines 25-31 * ---- | 1,3 | TECHNICAL FIELDS SEARCHED (Int. Cl.4) G 01 N 33/00 C 12 P 21/00 C 12 Q |

The present search report has been drawn up for all claims

| Place of search | Date of completion of the search | Examiner |
|---|---|---|
| VIENNA | 03-10-1989 | SCHNASS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document